# EUROPEAN PATENT SPECIFICATION

(11) **EP 2 082 028 B1**
(45) Date of publication and mention of the grant of the patent: **28.03.2018**
(21) Application number: 07734953.8
(22) Date of filing: 25.06.2007
(51) Int. Cl.: C12N 1/20, C12N 1/04, A23C 9/12, C12R 1/46

(54) **MICROBIAL LIQUID CULTURES HAVING HIGH STABILITY AND FERMENTATIVE ACTIVITY**
MIKROBIELLE FLÜSSIGKULTUREN MIT HOHER STABILITÄT UND GÄRUNGSAKTIVITÄT
CULTURES LIQUIDES MICROBIENNES PRÉSENTANT UNE GRANDE STABILITÉ ET UNE ACTIVITÉ FERMENTESCIBLE

(30) Priority: 27.09.2006 IT MI20061841
(43) Date of publication of application: 29.07.2009
(73) Proprietor: Mofin S.R.L., 28100 Novara (IT)
(72) Inventor: MOGNA, Giovanni, I-28100 Novara (IT); BRUNO, Federico, Frazione case Sparse I-28060 Granozzo con Monticello (IT)
(74) Representative: Hoffmann Eitle
(86) International application number: PCT/IB2007/001882
(87) International publication number: WO 2008/038075

(56) References cited:
- BARRETTE J ET AL: "The production of mixed cultures containing strains of Lactococcus lactis, Leuconostoc cremoris and Lactobacillus rhamnosus, on commercial starter media" JOURNAL OF INDUSTRIAL MICROBIOLOGY AND BIOTECHNOLOGY, vol. 25, no. 6, December 2000 (2000-12), pages 288-297, XP002463787 ISSN: 1367-5435
- HAMDI M ET AL: "Effect of Corn steep liquor supplementation and scale up on Lactococcus starter production" BIOPROCESS ENGINEERING, vol. 22, no. 1, January 2000 (2000-01), pages 23-27, XP002463788 ISSN: 0178-515X
- CHAMPAGNE C P ET AL: "STARTERS PRODUCED ON WHEY PROTEIN CONCENTRATES" MILCHWISSENSCHAFT, VV GMBH VOLKSWIRTSCHAFTLICHER VERLAG. MUNCHEN, DE, vol. 51, no. 10, 1996, pages 561-564, XP000678053 ISSN: 0026-3788
- PARENTE E ET AL: "GROWTH OF THERMOPHILIC STARTERS IN WHEY PERMEATE MEDIA1" JOURNAL OF DAIRY SCIENCE, AMERICAN DAIRY SCIENCE ASSOCIATION, SAVOY, IL, US, vol. 74, no. 1, January 1991 (1991-01), pages 20-28, XP000179739 ISSN: 0022-0302
- CHAMPAGNE C P ET AL: "Characteristics of lactococci cultures produced in commercial media" JOURNAL OF INDUSTRIAL MICROBIOLOGY, vol. 15, no. 6, 1995, pages 472-479, XP008087227 ISSN: 0169-4146
- YEZZI T L ET AL: "INCREASED NISIN IN CHEDDAR-TYPE CHEESE PREPARED WITH PH CONTROL OF THE BULK STARTER CULTURE SYSTEM" JOURNAL OF DAIRY SCIENCE, AMERICAN DAIRY SCIENCE ASSOCIATION, SAVOY, IL, US, vol. 76, no. 10, 1 October 1993 (1993-10-01), pages 2827-2831, XP000411298 ISSN: 0022-0302

## Description

The present invention relates to a method for preparing a liquid microbial culture, preferably with direct inoculation, characterized by a larger number of microbial cells per volume unit of culture than the one of similar known/traditional cultures, as well as by an increased stability and fermentative activity with respect thereto. Said culture is preferably a liquid starter culture.

The term starter culture refers to bacterial cultures comprising suitable microorganisms that are added to several types of food so as to improve their properties (e.g. nutritional, hygienic and organoleptic properties).

Starter cultures as are known at present (obtained with traditional methods, commonly used in fermentation technologies) are commercially available in a variety of forms: liquid cultures, dried cultures (usually by spray-drying), frozen cultures, freeze-dried cultures).

The method for obtaining the microbial biomass to be used as starter includes the development of the bacterial culture in a bioreactor or fermenter.

The way of growing microorganisms in the bioreactor that is most exploited industrially is the so-called batch processing (closed-system processing); in some cases only a continuous growth is used.

In batch culture, microorganisms are inoculated in the form of mother cultures into a given volume of liquid culture medium (varying depending on the bioreactor that is used), then thermally pre-treated at 85°C-122°C for about 15-40 minutes. Said culture medium is made up of various starting materials selected, depending on the microorganism to be developed, among: water, sources of nitrogen and/or carbon, mineral salts and growth activators, additives.

After inoculation, which is carried out at the optimal temperature for the growth of the species to be produced, the cells of the microorganism/s undergo a stage of exponential growth, after an initial latent period required for the synthesis of new enzymes and/or co-enzymes. During said growth stage, nutrients are consumed and growth products (biomass, metabolites) progressively accumulate. The nutritional environment inside the bioreactor is therefore subject to continuous variations, which lead in their turn to changes in cellular metabolism.

The above stage of exponential growth ends after some time and cells stop duplicating, basically for two reasons:
- exhaustion of one or more essential nutrients in the culture medium and/or
- accumulation up to inhibition levels of substances (metabolites) generated by the metabolism of said microorganisms.

Said metabolites are made up above all of organic acids, such as lactic acid and acetic acid, produced by sugar fermentation. The main effect is predominantly a progressive pH decrease of the cultural medium. The increase in hydrogen ion concentration involves first of all the cultural growth broth and then, after getting through the cellular membrane, also the bacterial cytoplasm. At the beginning the cell makes up for the inlet of hydrogen ions by means of a hydrogen ion pump designed to secrete H⁺ ions that are present in the cytoplasm. However, when cellular pH becomes too low, the hydrogen ion pump loses its effectiveness and intracellular hydrogen ion concentration tends to increase.

There are basically two consequences of this phenomenon. On the one hand, a further cellular growth is hindered and, on the other hand, the vitality of the biomass itself decreases, reducing as a result the fermentative activity for future application (e.g. during cheese-making). The microbial population thus reaches the so-called stationary stage, during which there is neither an increase nor a decrease in the number of cells.

A culture obtained with batch processing has a quite limited storability, depending on storage conditions and on the physiological state of the biomass at the end of the production process. A rapid refrigeration at the end of incubation can enable to store the starter also for 3-4 days without significant losses of vitality and activity.

The microbial load per volume unit of culture (or final cell concentration of the microorganism/s of the culture) characterizing liquid starter cultures known at present is of max. 0.5-1 billion cells/ml of culture (expressed as CFUs/ml, Colony Forming Units/ml, i.e. 0.5●10⁹-1●10⁹ CFUs/ml) but it is often not above 200 millions cells/ml of culture (2●10⁸ CFUs/ml).

The final stage in the preparation of a liquid starter culture includes packaging in adequately sterile environments of suitable volumes of culture (as a rule 50 liters) containing: water, the microbial biomass, the metabolites produced by said biomass during the growth stage, which have gradually accumulated in the culture medium, and residues of components of the culture medium.

Said liquid starter cultures are stored at a temperature of 3°C to 10°C, preferably of 3°C to 5°C. Therefore, also the distribution of liquid starter cultures should necessarily make use of the cold chain, which highly affects the cost of the finished product.

In the preparation of dehydrated (dried or freeze-dried) or frozen cultures, further stages are required, i.e.:
- separating the microbial biomass from the culture medium by way of a suitable isolation process (usually based on the use of centrifugation or filtration stages);
- washing said biomass in a suitable volume of a liquid, preferably isotonic medium, so as to remove the residues of the cultural medium;
- re-concentrating by filtration or centrifugation;
- dehydrating (usually by freeze-drying) or freezing after addition of suitable cryoprotectors.

Dehydrated cultures can be also be stored at room temperature for long periods and, as a result, the distribution thereof is easier. Anyhow, it is still preferred to store and distribute said cultures under refrigerated conditions, since this further extends their vitality.

The use of liquid starter cultures has the huge advantage of giving the final product (e.g. a dairy product) better organoleptic properties than those obtainable by using frozen or dehydrated cultures. As a matter of fact, the preparation of liquid starter cultures that are not liquid includes the removal from the microbial biomass of the metabolites that are present in the culture medium. However, said metabolites highly contribute to the development of flavor and, more generally, of the global organoleptic properties of the finished product: therefore, their presence in the starter is preferred.

Known liquid starter cultures can be applied by direct inoculation of the starting materials to be transformed, or they require some intermediate stages for the amplification of said culture so as to increase the bacterial population thereof (half-direct inoculation). Most liquid starter cultures fall into the second category and, therefore, require 1 to 4 stages of microbial reproduction. Said operation implies adequate knowledge and equipment so as to prevent possible contaminations with pollutants or phages, unbalances in the composition and/or loss of biological activity of the culture itself.

Therefore, the use of liquid starter cultures with direct inoculation seems to be advantageous in principle with respect to cultures with half-direct inoculation, which require longer times and additional equipment so as to carry out the stage of amplification of said culture.

In the dairy field, for instance, the necessary amount of liquid starter culture varies from one dairy product to another, depending on the inoculation envisaged for each type of cheese-making and to the cell density of the culture.

In general, inoculation enables to obtain in milk a concentration of vital cells of about 10-25 millions cells/ml (1●10⁷-2.5●10⁷ CFUS/ml). Since a commercial/traditional starter culture contains maximum 0.5●10⁹-1●10⁹ CFUS/ml, inoculation represents in volume 1% to 5% of the total milk volume. Therefore, 10-50 liters of traditional starter culture will be required for inoculating 1,000 liters of milk and then carrying out the necessary fermentative activity, which can be measured by way of pH decrease in milk as a function of time.

The previous example has shown that the volumes involved are quite high and, therefore, difficult to manage, above all as far as the distribution network is concerned.

Moreover, the lower storability (maximum 4-5 days, when culture storage temperature is of 3°C to 5°C) of known liquid starter cultures jeopardizes their ability to endure longer shipping times, thus limiting their use to areas that are closer to the production plant. The management of known liquid starter cultures, especially of those with direct inoculation, globally results in serious logistic problems due to the high volumes and low storability thereof.

For the reasons mentioned above, known liquid starter cultures have been replaced almost universally by dehydrated cultures and by frozen cultures. However, dairy products obtained with said cultures have less valid global organoleptic properties than products prepared using liquid starter cultures.

Therefore, there is still the need for starter cultures having at the same time the favorable characteristics of liquid starter cultures (especially in terms of better organoleptic properties of the products obtained therefrom) together with the highly easier global management characterizing frozen or dehydrated cultures.

Liquid starter cultures having the characteristics mentioned above are not known at present.

Barrette, J. et al (Journal of Industrial Microbiology and Biotechnology 2000, vol 25, n. 6. Pages 288-297) relates to the production of mixed cultures containing strains of *Lactococcus lactis, Leuconostoc cremoris* and *Lactobacillus rhamnosus* on commercial starter media.

Hamdi, M. et al (Bioprocess engineering 2000, vol 22, pages 23-27) describes the effect of corn steep liquor supplementation and scale up on *Lactococcus* starter production

Champagne, C.P. et al (Milchwissenschaft 1996, vol 51, 561-564) provides a comparison of milk to various whey protein concentrate media for the production of starters used in cheese manufacturing process.

Parente, E. et al (Journal of Dairy Science 1991, vol 74, pages 20-28) describes the growth of thermophilic starters in whey permeate media.

Champagne C. P. et *al* **(1995,** vol 15, pages 472-479) compares the characteristics of *Lactococci* cultures produced in commercial media and indicates that the protective effect of media obtained by a method including external pH control (EC) on culture stability would be limited.

The aim of the present invention is to answer ade-quately to the need referred to above. This aim and others, which shall be apparent from the following detailed description, have been achieved by the Applicant, who has unexpectedly found that a suitable control of the pH of the culture medium when producing the biomass allows to obtain a final liquid starter culture characterized by a larger number of cells than traditional liquid starter cultures.

Therefore, the present invention relates to the above liquid starter culture.

An object of the present invention is a method for producing said liquid starter culture, whose characteristics are disclosed in the appended independent claim.

The present invention further relates to the use of the above liquid starter culture for preparing industrial food products.

The present invention further relates to the use of said liquid starter culture in the dairy field. Preferred embodiments of the present invention are disclosed in the appended dependent claims. In the framework of the present invention, the term storability of a liquid starter culture refers to the lapse of time in which the concentration of vital microbial cell in said culture remains approximately constant.

The term cell concentration refers to the number of vital microbial cells (measured as colony-forming units or CFUs) per volume unit of culture.

Cell concentration is determined by way of one or more vital counts, generally on plates. Such method consists in determining the number of cells present in a fermentation medium that are able to form colonies on lab plates containing an adequate volume (generally 10 ml) of an agar medium. The fermentative activity of a liquid starter culture is determined by measuring pH decrease in time of a given volume of a suitable liquid, preferably milk, after inoculation with a given volume of said culture.

The storability of a liquid starter culture is related to maintaining in time the fermentative activity of said culture and depends on the average physiological state of the bacterial population on preparation. A liquid starter culture can be defined as valid/effective until its ability to sink the pH of a volume of a suitable liquid, generally milk, remains almost unchanged in time.

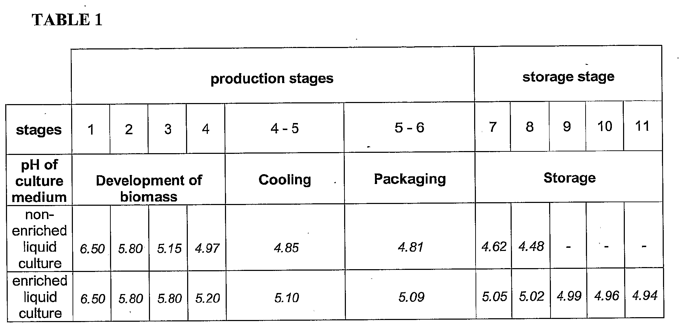

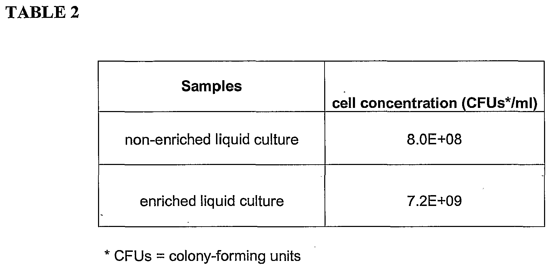

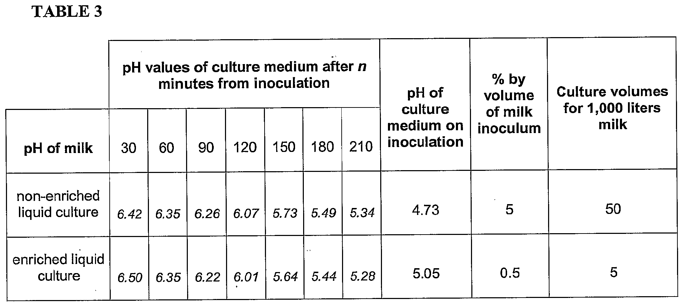

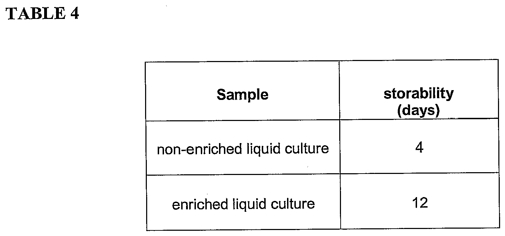

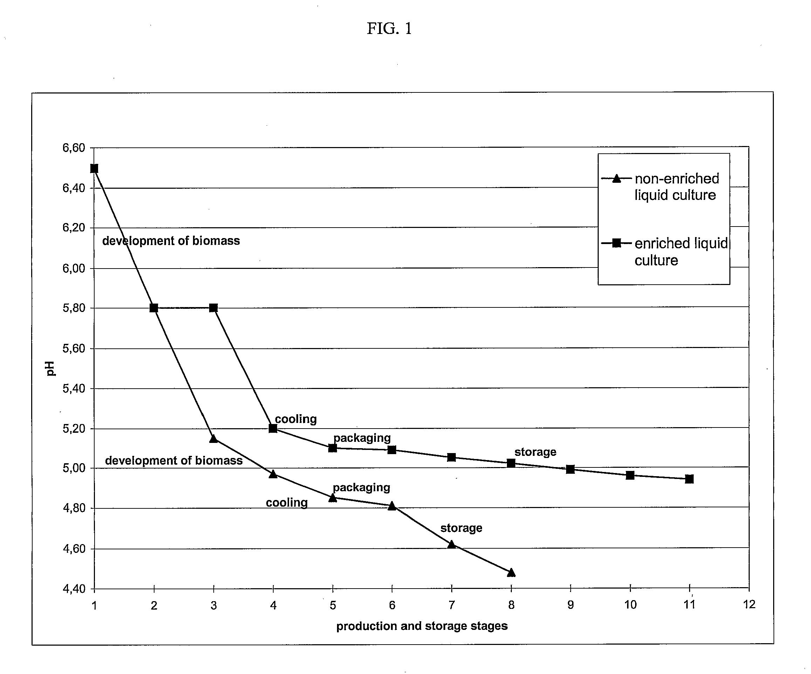

The characteristics and advantages of the present invention are pointed out in the following detailed description; moreover, they are further disclosed by way of example also in the accompanying Figures 1-4 and in the accompanying Tables 1-4 related to said figures, in which:
- **Fig. 1** is a graph of pH development in time of a known liquid starter culture (identified as "not enriched") and of a liquid starter culture according to the present invention (identified as "enriched"), both during production stage and during storage of said cultures; measurements have been carried out up to 6 days from packaging for the traditional culture, and up to 15 days from packaging for the enriched culture, respectively; storage for both cultures occurs at an average temperature of 4°C to 5°C;

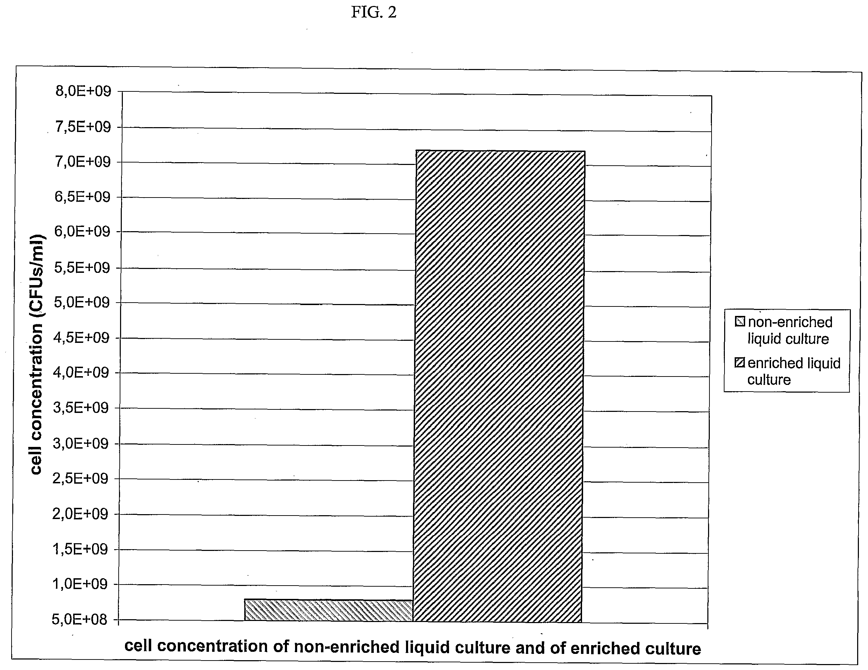

- **Fig. 2** contains the values of cell concentration of a traditional liquid starter culture (not enriched) and of a liquid starter culture according to the invention (enriched), respectively, obtained with corresponding production methods; said concentration values are expressed as CFUs/ml (the term nE+p on the ordinate corresponds to n●10^{p} CFUs/ml);

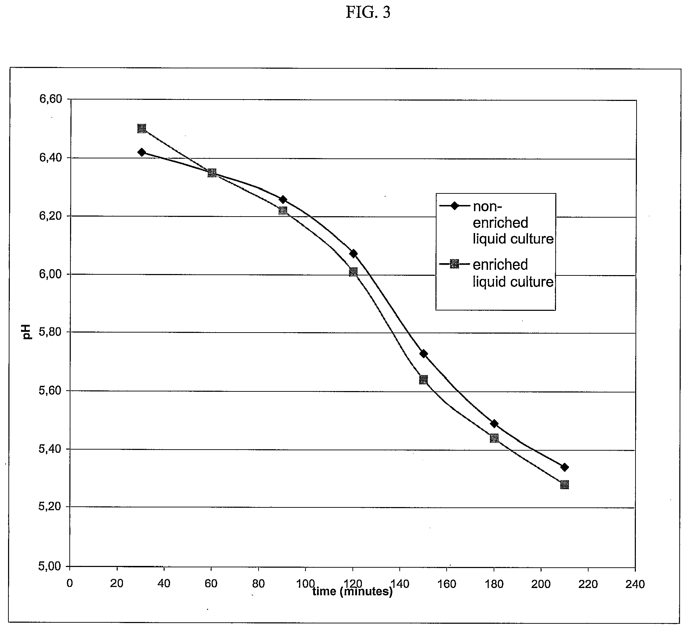

- **Fig. 3** shows a graph of the fermentative activity of a non-enriched liquid starter culture and of an enriched liquid starter culture according to the invention, respectively; said activities are expressed by showing the time development of pH decrease of a milk inoculated with said cultures; the non-enriched liquid starter culture has been inoculated to 5% by volume (V/V), whereas the enriched liquid starter culture has been inoculated to 0.5% by volume, i.e. 10 times less; the graph clearly points out that the enriched liquid starter culture according to the present invention shows the same fermentative activity as the known non-enriched culture at a dose that is 10 times lower (therefore, if the dose is the same, it is characterized by a fermentative activity that is 10 times higher) ;

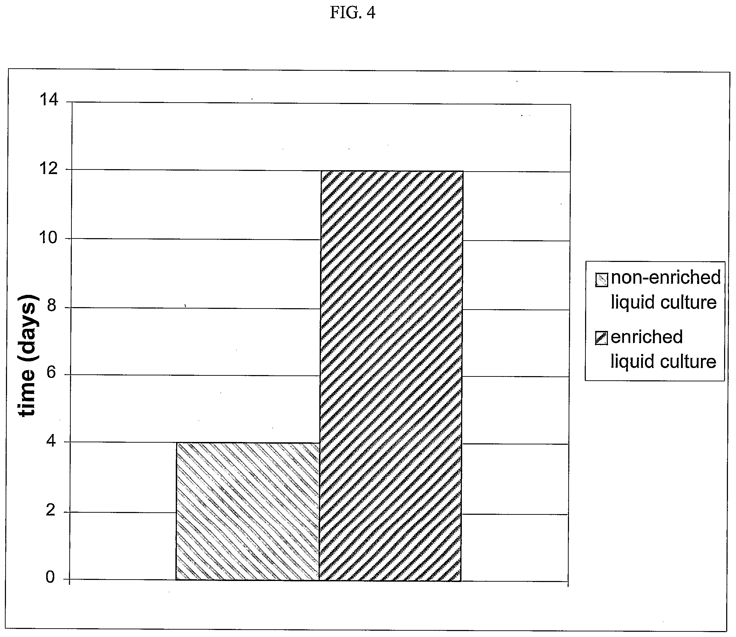

- **Fig. 4** shows the storability (expressed in days) of a non-enriched liquid starter culture and of an enriched liquid starter culture according to the invention; storability has been evaluated by measuring at successive times the fermentative activity of the culture;
- **Table 1** shows the pH values of the various production and storage stages that resulted in the graphs of Fig. 1;
- **Table 2** shows the values of cell concentration, expressed as CFUs/ml, that resulted in the histogram of Fig. 2;
- **Table 3** shows the pH values that resulted in Figure 3;
- **Table 4** shows data concerning culture stability, during storage at a temperature of 3°C to 5°C, that resulted in Fig. 4.

The present specification describes a liquid starter culture ("enriched") comprising at least one microorganism that is physiologically compatible with human and/or animal organisms, characterized in that the cell concentration of said microorganism in said culture (also referred to as "microbial charge") is higher than the maximum cell concentration of known liquid starter cultures. Said microorganism is chosen among bacterial strains having a probiotic valence.

The cell concentration of said liquid starter culture is as a rule >1.5 times the one of known liquid starter cultures. Said liquid culture has a cell concentration that is ≥2.5 times the concentration of known liquid starter cultures; preferably, said concentration is ≥6 times the one of known liquid starter cultures.

More preferably, said concentration is ≥10 times the one of known liquid starter cultures.

Therefore, the liquid starter culture is characterized by a cell concentration of >10⁹ CFUs/ml of culture; preferably, >1.5●109 CFUs/ml of culture. The above liquid starter culture is characterized by a cell concentration of >2.5●10⁹ CFUs/ml of culture, preferably, >6●10⁹ CFUs/ml. More preferably said concentration is of >10¹⁰ CFUs/ml.

Quite unexpectedly, the liquid starter culture obtained according to the present invention has proved to be characterized by an excellent storability.

The liquid starter culture obtained according to the present invention is characterized by a higher storability than known liquid starter cultures.

The above liquid starter culture obtained according to the method of the invention has a storability that is >1.5 times the storability of known liquid starter cultures; preferably, said storability is >2.5 times as much. More preferably, said storability is >4 times as against the storability of known liquid starter cultures. The liquid starter culture obtained according to the present invention, therefore, is characterized by a storability of ≥6 days, at an average storage temperature of 3°C to 5°C.

In a preferred embodiment of the invention, said liquid starter culture has a storability of ≥7.5 days; preferably, said storability is of ≥10 days. More preferably, said storability is of ≥13 days. Storability is always evaluated referring to an average storage temperature of 3°C to 5°C. Advantageously, the liquid storage culture obtained according to the present invention has a higher fermentative activity than known liquid starter cultures.

Said fermentative activity has proved to be on average at least 2 times as much as the one of known liquid starter cultures. Said liquid starter culture has a fermentative activity 4 to 35 times as much as the fermentative activity of known liquid starter cultures; preferably, said activity is 8 to 30 times as much. More preferably, said fermentative activity is 12 to 25 times as much as the activity of known liquid starter cultures.

The liquid starter culture is preferably a culture with direct inoculation.

The method for obtaining an enriched liquid starter culture according to the present invention includes at least a step consisting in controlling the pH of the culture medium. Said pH control step can apply both to a batch and to a continuous growth of the microbial biomass.

During said control step, pH is kept as a rule within a range of 4.5 to 7.0; preferably said pH range is of 5.5 to 6.5; more preferably, of 5.6 to 6.2.

In a preferred embodiment, pH is of 5.7 to 6.0.

In order to perform pH control as described above, the Applicant has found it useful to add to the culture medium for growing microorganisms at least one base or a suitable mixture of bases.

Preferably, said at least one base is added to the culture medium for growing microorganisms progressively, so as to enable pH to be kept within the values referred to above for the time required for obtaining the desired degree of growth of the microorganism/s.

The base is chosen from the group comprising: carbonate ion, in mono- and/or dibasic forms, ammoniac, hydroxides, e.g. ammonium, sodium, potassium hydroxides and others, phosphate ion, in mono- and/or di- and/or tribasic forms, sulfate ion, in mono- and/or dibasic forms, citrate ions, tartrate ion, other bases that are physiologically compatible with culture microorganisms, and/or mixtures thereof.

In a preferred embodiment, the base used to keep pH values of the culture medium approximately constant is carbonate ion, in mono- and/or dibasic forms; said carbonate ion derives from any suitable source thereof: preferably from the dissolution of calcium carbonate.

In another preferred embodiment, the base used is ammoniac and/or ammonium hydroxide.

The base neutralizes hydrogen ions deriving from the dissociation of organic acids forming during the fermentation of the various organic substrates thanks to the microbial biomass.

Said step of controlling the pH of the culture medium is preferably started automatically when the culture medium reaches a pre-established pH threshold, after a lapse of time depending on the type of growing microorganism and on the culture conditions used.

The duration of the pH control step (i.e. the time during which said at least one base is progressively added to the culture medium) is extended for a time generally corresponding to the one required by the microorganism to undergo 2 to 5 cell divisions or cell duplications, during the stage of exponential growth.

The time required for a cell duplication to occur varies depending on the type of microorganisms and on the culture conditions used for said microorganism. Said duplication time is determined a priori for every single microorganisms with known methods, commonly used by all skilled technicians.

Once said pH control step is over, said pH is let sink spontaneously and gradually for a time 0.5 to 1 times as much as the time required for the microorganism/s to undergo a cell duplication. The final pH value thus obtained varies from species to species.

The final pH of the liquid starter culture thus obtained is generally of 4.7 to 5.6; preferably, it is of 5.0 to 5.2.

Now the final culture containing water, the microbial biomass, the metabolites produced by said biomass during the growth stage, and the residues of components of the culture medium, if present, is cooled to a temperature of 4°C to 8°C so as to obtain the finished product, i.e. the enriched liquid starter culture according to the present invention.

The enriched liquid starter culture thus obtained is directly packaged and stored at 3-5°C, before being sent to the commercial biotechnological use for which it has been produced.

With the method of controlling the pH of the culture medium as described above, it has been possible to obtain, advantageously and in a repeatable manner, a higher development of the biomass in the environment of the bioreactor, a higher vitality, a higher storability, an increased fermentative activity of said biomass.

Still advantageously, the starter culture obtained with the method according to the present invention has proved to be basically without residual sugars. As a matter of fact, it has been found that, during the stage of exponential growth in accordance with the method of the present invention, the sources of carbon and/or of energy (in particular of sugars), which are present as starting materials making up the culture medium, are exploited almost completely. Such situation enables to highly reduce (if not to eliminate completely) the so-called post-acidification stage, which in known starter cultures takes place spontaneously after packaging the culture, just because of the presence of significant residual amounts of said sugars. Said post-acidification stage negatively affects the stability of the finished product due to the use of the residual sugars by the biomass, thus resulting in a further production of organic acids and to a further pH decrease of the liquid starter culture. Post-acidification, which occurs unavoidably in known liquid starter cultures, therefore reduces the pH of the culture up to values (varying depending on the microbial species) of 4.0 to 4.6, sometimes even below 3.8, thus negatively affecting the vitality and storability of said culture.

The unexpected substantial elimination of the post-acidification stage has proved to be one of the possible factors contributing to the improvement of stability of the finished product.

By way of absolutely non-limiting example of embodiment, the following discloses a general method for preparing an enriched liquid starter culture, wherein said method preferably includes the following steps:
a) decontaminating the bioreactor by flowing vapor; preferably for 30 minutes;
b) thermally treating the culture medium in the reactor; preferably at 85C°-90°C for 20-30 minutes;
c) cooling the culture medium up to the temperature of inoculation of the microorganism used;
d) inoculating the culture medium as in step c) with an effective amount of mother culture of at least one bacterial microorganism, or of a mixture of microorganisms;
e) waiting for the beginning of fermentation and for the beginning of the stage of exponential growth of the bacterial biomass, for a time depending on the type/s of microorganism/s used;
f) controlling the pH of the culture medium, keeping said value in a range of 5.7 to 6.0, by way of progressive additions of ammonium hydroxide and/or carbonate, preferably for the time required for 2-5 cell duplications of the microorganism/s to occur;
g) letting the pH of the culture medium sink spontaneously to a value of 5.0 to 5.2; preferably in a lapse of time sufficient for a further cell duplication to occur;
h) cooling the culture as in step g) up to a temperature of 4°C to 8°C;
i) packaging said culture as in step h) under sterile conditions.

In a preferred embodiment, the culture medium as in step b) comprises (amounts referred to one liter of culture medium): casein peptones 10 g; meat extract, 5 g; yeast extract, 10 g; MgSO₄, 200 mg; dextrose, 20 g; Tween 80, 1 ml; water q.s. to 1 1 of final culture medium.

In particular cases, the thermal treatment of the culture medium as in step b) can be carried out at 121°C for 15 min.

The inoculation temperature as in step c) can take average values of 18°C to 47°C, depending on the microorganism used, on the corresponding growth conditions required by said microorganism, and on the amount of inoculum used.

For instance, for mesophilic microorganisms, said temperature is preferably of about 18°C to about 32°C; for thermophilic microorganisms, said temperature is preferably of about 37°C to about 45°C.

The percentage of inoculum (V/V) as in step d) can vary from 0.5% to 10%, depending on the microorganism/s to be grown, on the characteristics of the culture medium, on the characteristics of development of the microbial biomass.

Said inoculum is obtained by preparing in a suitable bioreactor with known methods a mother culture of the desired microorganism/s.

Said mother culture is prepared in its turn starting from a sample, deriving from a cell library, by successive sub-cultures or amplifications, in progressively increasing volumes of medium.

Said mother culture can be used also in dehydrated form, if volumes of fermentation medium on average below about 10,000 liters are used. In this case the inoculum is preferably such as to provide on average 10-25●10⁶ CFUs/ml to the inoculated culture medium.

The mother culture thus obtained is then inoculated and let develop in the above-described culture medium. Preferably, measures apt to prevent as much as possible contamination risks are taken for the bioreactor: air getting into the fermentation compartment is filtered by way of sterile filters; the compartment itself is sterilized with high-temperature vapor between one production cycle and the following one; also electrodes used for pH control and temperature sensors are sterilized; biomass production takes place under sterile air or nitrogen overpressure conditions.

In a particularly preferred embodiment, the first packaging of the enriched liquid starter culture as described above aims also at reducing almost completely the risks of contamination for the product, and occurs in specific environments under a laminar flow of sterile air.

Preferably, packaging is carried out in 3 to 5 liter polyethylene/aluminum bags, which are filled up with a peristaltic pump and autoclavable silicone pipes, then sterilized and connected directly to the bioreactor. Bags are also welded under sterile conditions. For the storage of the liquid starter culture according to the present invention after packaging, the latter is preferably kept at a temperature of 0-100C; more preferably of 3-5°C.

The liquid starter culture obtained by the method according to the invention comprises at least one microbial strain (or a mixture of more microbial strains) chosen from the groups comprising the genus: *Lactobacillus, Bifidobacterium, Lactococcus, Pediococcus, Leuconostoc, Streptococcus, Enrerococcus.*

Preferably, the following species of the genus Lactobacillus have been used: *L. pentosus, L. plantarum, L. casei, L. casei ssp. paracasei, L. casei* ssp. *rhamnosus, L. acidophilus, L. delbrueckii* ssp. *bulgaricus, L. fermentum, L. gasseri.*

Preferably, the following species of the genus Bifidobacterium have been used: *B. longum, B. breve, B. lac-tis, B. adolescentis, B. pseudocatenulatum, B. catenulatum.*

Preferably, the following species of the genus *Lactococcus* have been used: *L. lactis* and *L. lactis* ssp. *lactis.* Preferably, the species *S. thermophilus* of the genus *Streptococcus* has been used.

-Among the species mentioned above, the bacterial strains listed in the following Table 5 have proved to be particular preferred.

### TABLE 5 (Staphylococcus xylosus not part of the claimed invention)

**TABLE 5**

| **No.** | **Name** | **Number of** deposit | **Date of deposit** | **Depositor** |
|---|---|---|---|---|
| 1 | *Streptococcus thermophilus* | LMG P-18383 | 5.05.1998 | ANIDRAL S.R.L. |
| 2 | *Streptococcus thermophilus* | LMG P-18384 | 5.05.1998 | ANIDRAL S.R.L. |
| 3 | *Lactobacillus pentosus* | LMG P-21019 | 16.10.2001 | Laboratorio Microbiologico Grana Provolone SRL |
| 4 | *Lactobacillus plantarum* | LMG P-21020 | 16.10.2001 | Laboratorio Microbiologico Grana Provolone SRL |
| 5 | *Lactobacillus plantarum* | LMG P-21021 | 16.10.2001 | Laboratorio Microbiologico Grana Provolone SRL |
| 6 | *Lactobacillus plantarum* | LMG P-21022 | 16.10.2001 | Laboratorio Microbiologico Grana Provolone SRL |
| 7 | *Lactobacillus plantarum* | LMG P-21023 | 16.10.2001 | Laboratorio Microbiologico Grana Provolone SRL |
| 8 | *Lactobacillus casei ssp. Paracasei* | LMG P-21380 | 31.01.2002 | ANIDRAL S.R.L. |
| 9 | *Lactobacillus belonging to the acidophilus group* | LMG P-21381 | 31.01.2002 | ANIDRAL S.R.L. |
| 10 | *Bifidobacterium longum* | LMG P-21382 | 31.01.2002 | ANIDRAL S.R.L. |
| 11 | *Bifidobacterium breve* | LMG P-21383 | 31.01.2002 | ANIDRAL S.R.L. |
| 12 | *Bifidobacterium lactis* | LMG P-21384 | 31.01.2002 | ANIDRAL S.R.L. |
| 13 | *Lactobacillus plantarum* | LMG P-21385 | 31.01.2002 | MOFIN S.R.L. |
| 14 | *Lactococcus lactis ssp. lactis* | LMG P-21387 | 15.03.2002 | MOFIN S.R.L. |
| 15 | *Lactococcus lactis* | LMG P-21388 | 31.01.2002 | MOFIN S.R.L. |
| | *ssp. lactis* | | | |
| 16 | *Lactobacillus plantarum* | LMG P-21389 | 15.03.2002 | MOFIN S.R.L. |
| 17 | *Streptococcus thermophilus* | DSM 16506 | 18.06.2004 | ANIDRAL S.R.L. |
| 18 | *Streptococcus thermophilus* | DSM 16507 | 18.06.2004 | ANIDRAL S.R.L. |
| 19 | *Bifidobacterium longum* | DSM 16603 | 20.07.2004 | ANIDRAL S.R.L. |
| 20 | *Bifidobacterium breve* | DSM 16604 | 20.07.2004 | ANIDRAL S.R.L. |
| 21 | *Lactobacillus casei ssp. rhamnosus* | DSM 16605 | 20.07.2004 | ANIDRAL S.R.L. |
| 22 | *Lactobacillus delbrueckii ssp. bulgaricus* | DSM 16606 | 20.07.2004 | ANIDRAL S.R.L. |
| 23 | *Lactobacillus delbrueckii ssp. bulgaricus* | DSM 16607 | 20.07.2004 | ANIDRAL S.R.L. |
| 24 | *Streptococcus thermophilus* | DSM 16590 | 20.07.2004 | ANIDRAL S.R.L. |
| 25 | *Streptococcus thermophilus* | DSM 16591 | 20.07.2004 | ANIDRAL S.R.L. |
| 26 | Streptococcus *Thermophilus* | DSM 16592 | 20.07.2004 | ANIDRAL S.R.L. |
| 27 | *Streptococcus thermophilus* | DSM 16593 | 20.07.2004 | ANIDRAL S.R.L. |
| 28 | Bifidobacterium *adolescentis* | DSM 16594 | 21.07.2004 | ANIDRAL S.R.L. |
| 29 | *Bifidobacterium adolescentis* | DSM 16595 | 21.07.2004 | ANIDRAL S.R.L. |
| 30 | *Bifidobacterium breve* | DSM 16596 | 21.07.2004 | ANIDRAL S.R.L. |
| 31 | *Bifidobacterium pseudocatenulatum* | DSM 16597 | 21.07.2004 | ANIDRAL S.R.L. |
| 32 | *Bifidobacterium pseudocatenulatum* | DSM 16598 | 21.07.2004 | ANIDRAL S.R.L. |
| 33 | *Staphylococcus xylosus* | DSM 17102 | 01.02.2005 | ANIDRAL S.R.L. |
| 34 | *Bifidobacterium adolescentis* | DSM 17103 | 01.02.2005 | ANIDRAL S.R.L. |
| 35 | *Lactobacillus* plantarum | DSM 17104 | 01.02.2005 | ANIDRAL S.R.L. |
| 36 | *Streptococcus thermophilus* | DSM 17843 | 21.12.2005 | ANIDRAL S.R.L. |
| 37 | *Streptococcus thermophilus* | DSM 17844 | 21.12.2005 | ANIDRAL S.R.L. |
| 38 | *Streptococcus Thermophilus* | DSM 17845 | 21.12.2005 | ANIDRAL S.R.L. |
| 39 | *Lactobacillus fermentum* | DSM 18295 | 24.05.2006 | ANIDRAL S.R.L. |
| 40 | *Lactobacillus fermentum* | DSM 18296 | 24.05.2006 | ANIDRAL S.R.L. |
| 41 | *Lactobacillus fermentum* | DSM 18297 | 24.05.2006 | ANIDRAL S.R.L. |
| 42 | *Lactobacillus fermentum* | DSM 18298 | 24.05.2006 | ANIDRAL S.R.L. |
| 43 | *Lactobacillus gasseri* | DSM 18299 | 24.05.2006 | ANIDRAL S.R.L. |
| 44 | *Lactobacillus gasseri* | DSM 18300 | 24.05.2006 | ANIDRAL S.R.L. |
| 45 | *Lactobacillus gasseri* | DSM 18301 | 24.05.2006 | ANIDRAL S.R.L. |
| 46 | *Lactobacillus gasseri* | DSM 18302 | 24.05.2006 | ANIDRAL S.R.L. |
| 47 | *Bifidobacterium Adolescentis* | DSM 18350 | 15.06.2006 | ANIDRAL S.R.L. |
| 48 | *Bifidobacterium adolescentis* | DSM 18351 | 15.06.2006 | ANIDRAL S.R.L. |
| 49 | *Bifidobacterium adolescentis* | DSM 18352 | 15.06.2006 | ANIDRAL S.R.L. |
| 50 | *Bifidobacterium catenulatum* | DSM 18353 | 15.06.2006 | ANIDRAL S.R.L. |
| 51 | *Streptococcus thermophilus* | DSM 18613 | 13.09.2006 | MOFIN S.R.L. |
| 52 | *Streptococcus thermophilus* | DSM 18614 | 13.09.2006 | MOFIN S.R.L. |
| 53 | *Streptococcus Thermophilus* | DSM 18615 | 13.09.2006 | MOFIN S.R.L. |
| 54 | *Streptococcus thermophilus* | DSM 18616 | 13.09.2006 | MOFIN S.R.L. |
| 55 | *Streptococcus thermophilus* | DSM 18617 | 13.09.2006 | MOFIN S.R.L. |
| 56 | *Streptococcus thermophilus* | DSM 18618 | 13.09.2006 | MOFIN S.R.L. |
| 57 | *Streptococcus thermophilus* | DSM 18619 | 13.09.2006 | MOFIN S.R.L. |
| 58 | *Streptococcus thermophilus* | DSM 18620 | 13.09.2006 | MOFIN S.R.L. |
| 59 | *Streptococcus thermophilus* | DSM 18621 | 13.09.2006 | MOFIN S.R.L. |
| 60 | *Streptococcus thermophilus* | DSM 18622 | 13.09.2006 | MOFIN S.R.L. |
| 61 | *Streptococcus thermophilus* | DSM 18623 | 13.09.2006 | MOFIN S.R.L. |
| 62 | *Streptococcus thermophilus* | DSM 18624 | 13.09.2006 | MOFIN S.R.L. |
| 63 | *Streptococcus Thermophilus* | DSM 18625 | 13.09.2006 | MOFIN S.R.L. |

Among the bacterial strains listed in Table 5, those from number 51 to number 63, belonging to the genus *Streptococcus,* species *thermophilus,* are new and have been deposited at DSMZ (*"Deutsche Sammlung von Mikroorganismen* und *Zellkulturen GmbH"*: Inhoffenstr. 7B, D-38124 Braunschweig, Germany) on 13.09.2006 by Mofin S.r.l., Via Pietro Custodi 12 - 28100 NOVARA, under access numbers: DSM 18613; DSM 18614; DSM 18615; DSM 18616; DSM 18617; DSM 18618; DSM 18619; DSM 18620; DSM 18621; DSM 18622; DSM 18623; DSM 18624; DSM 18625, respectively.

### Characterization of the new strains.

The new strains in Table 5 (those between number 51 and number 63) have been isolated from various samples of cow's milk and of natural starters, after suitable treatment as commonly provided for by the specific known technique.

### Growing method:

medium M17 broth according to Terzaghi (Merck ref. 1.15029);
sterilization: 15 min. at 121°C;
pH before sterilization = 7.35 ± 0.5;
pH after sterilization = 7.01 ± 0.5;
incubation: 4h at 44°C;
storage: -25°C;
vitality test: acidification curve in milk at 44°C.
Description:
   they have the appearance of spherical cells in short chains; optional anaerobes; gram-positive; catalasis-negative; they do not grow at 10°C with 40% bile and 6.5% NaCl; growth at 45°C; they do not give rise to β-hemolysis; they hydrolyze arginine and esculin; they produce L-lactic acid; final pH after growth in a medium containing glucose as source of energy = 4.0-4.5; final pH after growth in milk = 4.3-4.5.

As far as known strains listed in Table 5 are concerned, they have preferably been isolated (depending on the type of strain) from samples of animal milk according to techniques known in the field; or (especially as far as probiotic strains are concerned) they have preferably been isolated from human fecal material according to techniques known in the field (if necessary, also using intestinal brushing techniques). For instance, the strains belonging to the genus *Streptococcus,* species *thermophilus,* referred to with numbers 1, 2, 17, 18, 24-27, 36-38 in Table 5, have the same characteristics as those mentioned above.

The strains belonging to the genus *Bifidobacterium* (referred to with numbers 10-12, 19, 20, 28-32, 34, 47-50 in Table 5) are characterized by:
growth in Trypticase-phytone-yeast extract medium (TPY) at 37°C;
they have the appearance of rods of various shapes; gram-positive; not rapid in acid production; they do not form spores; not movable; anaerobic; they degrade glucose only by way of fructose-6-phosphate shunt.

In particular, the strains referred to with numbers 28-32 in Table 5 are characterized in that they produce folic acid.

The strain belonging to the genus *Lactobacillus* species *casei* ssp. *rhamnosus* (referred to with number 21 in Table 5) is characterized by:
growth in culture broth MRS (DIFCO, ref. 288130) at 37°C;
it has the appearance of rods, often with square ends and tending to form chains; optionally heterofermenting; it produces L-lactic acid; able to grow at temperatures of 15°C to 45°C; it has a natural resistance to vancomycin.

The strains belonging to the genus *Lactobacillus* species *delbrueckii* ssp. *bulgaricus* (referred to with numbers 22, 23 in Table 5) are characterized by: growth in culture broth MRS (DIFCO, ref. 288130) at 37°C;
they have the appearance of rods with rounded ends, both single and in short chains; they grow well at 45°C; they do not generate spores; gram-positive; compulsorily homofermenting; they produce D-lactic acid; they ferment fructose, glucose and lactose only.

The strains belonging to the genus *Lactobacillus,* species *plantarum* (referred to with numbers 4-7, 13, 16, 35 in Table 5) are characterized by:
growth in culture broth MRS (DIFCO, ref. 288130) at 30°C;
they have the appearance of short rods, both single and in short chains; they grow well at 30°C; they do not generate spores; gram-positive; optionally heterofermenting.

The strains belonging to the genus *Lactobacillus,* species *fermentum* (referred to with numbers 39-42 in Table 5) are characterized by:
origin: from intestinal brushing;
growth in culture broth MRS (DIFCO, ref. 288130) at 37°C;
they have the appearance of short rods; they grow well at 37°C to 45°C; compulsorily heterofermenting; they produce DL-lactic acid.

The strains belonging to the genus *Lactobacillus,* species *gasseri* (referred to with numbers 43-46 in Table 5) are characterized by:
origin: from intestinal brushing;
growth in culture broth MRS (DIFCO, ref. 288130) at 37°C;
they have the appearance of rods with rounded ends, both single and in chains; they grow well at 37°C to 45°C; compulsorily homofermenting; they produce DL-lactic acid. The strain belonging to the genus *Staphylococcus,* species *xylosus* (referred to with number 33 in Table 5, not part of the present invention) is characterized by:
   growth in culture broth M17 according to Terzaghi (Merck ref . 15029) at 37°C;
   it has the appearance of spheres with a diameter 0 = 0.8-1.2 µm; as single or double cells or as tetrads; optional anaerobe; optimal growth under aerobic conditions at 25-350C; good growth at NaCl concentrations up to 10%; producer of catalase; if reduces nitrates and has the activity as alkaline phosphatase.

The other strains of Table 5 have proved to have similar characteristics with respect to those of the strains belonging to the same genus.

The liquid culture obtained according to the present invention is advantageously used as starter for preparing industrial food products (e.g. dairy products such as cheese, yogurts, fermented milks; bread, baked products, salamis and sausages in general, alcoholic drinks).

In a preferred embodiment, said liquid starter culture is used for preparing dairy products; the dairy prod-, ucts thus obtained have at least all the organoleptic properties that can be obtained with known liquid starter cultures.

The inoculation of milk in a boiler with an enriched liquid starter culture is on average below 1% by volume (V/V) with respect to the total volume of milk to be treated; preferably, said inoculum is of 0.2% to 0.8% (V/V) with respect to milk; more preferably, it is of 0.3% to 0.6% (V/V); advantageously, it is of ≤0.5% (V/V).

Therefore, the volume of milk to be inoculated being the same, a smaller volume of enriched liquid starter culture is sufficient. For the inoculation of 1,000 liters of milk, for instance, 3 to 6 liters of starter culture are advantageously sufficient, instead of 10-50 liters required for the inoculation of the same amount of milk with a known liquid starter culture. Such volume of enriched liquid starter culture corresponds - in terms of fermentative yield, which can be measured by way of the decrease of pH values in milk as a function of time- to 10-50 liters of a known commercial liquid starter culture.

The higher storability of the enriched liquid starter culture enables to reduce the frequency with which said culture is distributed to dairies, as well as to cover on the whole longer distances and shipping times without loss of the fermentative activity, both in qualitative and in quantitative terms, that is necessary to ensure the characteristics of appearance, taste and aroma of the final dairy product.

By way of absolutely non-limiting example, the following discloses the preparation of some particularly preferred liquid starter cultures according to the invention

Example 1 - Liquid starter culture comprising the bacterial strain *Streptococcus thermophilus* DSM 18613. Following the procedure of the general method of preparation described above, using as base a 25% solution of ammonium hydroxide, 10,000 1 of liquid starter culture for dairy use have been obtained with batch processing, having a final concentration of *Streptococcus thermophilus* DSM 18613 corresponding to 7.2●10⁹ CFUs/ml of culture.

Said culture, stored at a temperature of 3°C to 5°C, has proved to be stable for more than 12 days. Following the same method of preparation, liquid starter cultures have also been prepared comprising a bacterial strain selected among:
a) *Lactobacillus delbrueckii* ssp. *bulgaricus* DSM 16606;
b) *Lactobacillus casei* ssp. *rhamnosus* DSM 16605;
c) *Lactobacillus plantarum* LMG P-21020;
d) *Lactococcus lactis* ssp. *lactis* LMG P-21388;
f) *Bifidobacterium longum* LMG P-21382;
Said cultures, stored at a temperature of 2°C to 4°C, have proved to be stable for more than 12 days. Cell concentration of the cultures is of 3●10⁹ to 10●10⁹ CFUs/ml of culture medium.

Example 2 - Liquid starter culture comprising a mixture of the following bacterial strains: *Streptococcus thermophilus* DSM 16506; *Lactobacillus delbrueckii* ssp. *bulgaricus* DSM 16606.

Following the procedure of the general method of preparation described above, a culture medium made up of (amounts referred to one liter of culture medium): rice peptone, 10 g; yeast extract, 10 g; yeast hydrolysate, 10 g; dextrose, 20 g; MgSO₄, 200 mg; K₂HPO₄, 7.975 g; NaH₂PO₄, 1.437 g; MgSO₄●7H₂O, 50 mg; Tween 80, 1 ml, has bee inoculated with mother cultures of *Streptococcus thermophilus* DSM 16506 and *Lactobacillus delbrueckii* ssp. *bulgaricus* DSM 16606 in a mutual ratio of cell concentration of 1:1.

During the growth of the biomass, pH is controlled as described above by addition of calcium carbonate.

8,000 liters of liquid starter culture for dairy use have been obtained, having a final cell concentration of *Streptococcus thermophilus* DSM 16506 corresponding to 5.6●10⁹ CFUs/ml of culture, and of *Lactobacillus delbrueckii* ssp. *bulgaricus* DSM 16606 corresponding to 1.5●10⁹ CFUs/ml of culture.

Said culture, stored at a temperature of 3°C to 5°C, has proved to be stable for more than 12 days.

## Claims

1. A method for preparing a liquid starter culture comprising at least one physiologically compatible microorganism, **characterized in that** the cell concentration of said microorganism in said liquid starter culture is of >10⁹ CFUs/ml of culture medium, said liquid starter culture has storability of 6 days or more, at an average temperature of 3°C to 5°C and is basically without residual sugars,
wherein said method includes
**i)** inoculating a culture medium with an effective amount of mother culture of at least one bacterial strain, or of a mixture of strains;
**ii)** waiting for the beginning of fermentation and for the beginning of the stage of exponential growth of the bacterial biomass;
**iii)** controlling the pH of the culture medium, keeping said value in a range of 4.5 to 7.0, by way of progressive additions of at least one base or a mixture of more bases, for the time required for 2 to 5 cell duplications of the strain/s to occur;
**iv)** letting the pH of the culture medium sink spontaneously to a value of 4.7 to 5.6, in a period of time sufficient for a further cell duplication to occur;
**v**) cooling the culture obtained in step iv) down to a temperature of 4°C to 8°C;
wherein said at least one microorganism is chosen from the group of microbial strains of one of the genera:
*Lactobacillus, Leuconostoc, Bifidobacterium, Lactococcus, Pediococcus, Streptococcus* and *Enterococcus.*

2. The method according to claim 1, wherein in the step iii) during said control step the pH value is kept within a range of 5.6 to 6.2.

3. The method according to claim 2, wherein in the step iii) the pH value is kept within a range of 5.7 to 6.0.

4. The method according to any one of the claims 1 to 3, wherein said at least one base is chosen from the group comprising: carbonate ion, in mono- and/or dibasic forms, ammonia, hydroxides, e.g. ammonium, sodium, potassium hydroxides, phosphate ion, in mono- and/or di- and/or tribasic forms, sulfate ion, in mono- and/or dibasic forms, citrate ions, tartrate ion, and/or bases that are physiologically compatible with microorganisms of the culture, and/or mixtures thereof.

5. The method according to claim 4, wherein said step of pH control is carried out by adding to the culture medium for growing the bacterial strain/s ammonia and/or ammonium hydroxide, or carbonate ion, in mono- and/or dibasic forms.

6. The method according to claim 1, wherein the final pH of the culture is of 5.0 to 5.2.

7. The method according to any of the preceding claims, wherein:
- said strains of the genus *Lactobacillus* are chosen from the following species: *L. pentosus, L. plantarum, L. casei, L. casei ssp. paracasei, L. casei* ssp. *rhamnosus, L. acidophilus, L. delbrueckii* ssp. *bulgaricus, L. fermentum, L. gasseri;*
- said strains of the genus *Bifidobacterium* are chosen from the following species: *B. longum, B. breve, B. lactis, B. adolescentis, B. pseudocatenulatum, B. catenulatum;*
- said strains of the genus *Lactococcus* are chosen from the following species: *L. lactis* and *L. lactis* ssp. *lactis;*
- said strains of the genus *Streptococcus* are chosen from the species *S. thermophilus.*

## Patentansprüche

1. Verfahren zur Herstellung einer flüssigen Starterkultur mit mindestens einem physiologisch kompatiblen Mikroorganismus, **dadurch gekennzeichnet, dass** die Zellkonzentration dieses Mikroorganismus in der flüssigen Starterkultur >10⁹ KBE/ml im Nährboden beträgt, wobei die flüssige Starterkultur sechs Tage oder länger bei einer Durchschnittstemperatur von 3°C bis 5°C gelagert werden kann und nahezu ohne Restzucker ist, wobei dieses Verfahren beinhaltet:
**i)** Inokulation eines Nährmediums mit einer wirksamen Menge einer Mutterkultur mindestens eines Bakterienstammes oder einer Mischung von Stämmen;
**ii)** das Warten auf den Beginn der Fermentierung und des exponentiellen Wachstumsstadiums der Bakterienbiomasse;
**iii)** die Kontrolle des pH-Wertes des Nährmediums, die Aufrechterhaltung dieses Wertes zwischen 4,5 bis 7,0 durch die fortlaufende Hinzufügung von mindestens einer Base oder eines Gemisches von Basen während eines Zeitraums, der für den Vorgang von 2 bis 5 Zellduplikationen des Stammes bzw. der Stämme notwendig ist;
**iv)** das spontane Sinkenlassen des pH-Wertes des Nährmediums auf einen Wert von 4,7 bis 5,6 während eines Zeitraums, der für den Vorgang einer weiteren Zellduplikation ausreichend ist;
**v)** Abkühlen der in Schritt iv) erhaltenen Kultur auf eine Temperatur von 4°C bis 8°C;
wobei der mindestens eine Mikroorganismus aus der Gruppe der mikrobiellen Stämme einer der folgenden Gattungen ausgewählt wird:
*Lactobacillus, Leuconostoc, Bifidobacterium, Lactococcus, Pediococcus, Streptococcus* und *Enterococcus.*

2. Verfahren nach Anspruch 1, wobei in Schritt iii) während des Kontrollschritts der pH-Wert auf 5,6 bis 6,2 gehalten wird.

3. Verfahren nach Anspruch 2, wobei in Schritt iii) der pH-Wert auf 5,7 bis 6,0 gehalten wird.

4. Verfahren nach einem der Ansprüche 1 bis 3, wobei die mindestens eine Base ausgewählt wird aus einer Gruppe enthaltend: Carbonat-Ionen in ein- und/oder zweibasischen Formen, Ammoniak, Hydroxide, z.B. Ammonium-, Natrium- oder Kalium-Hydroxide, Phosphat-Ionen in ein- und/oder zwei- und/oder dreibasischen Formen, Sulfat-Ionen in ein- und/oder zweibasischen Formen, Citrat-Ionen, Tartrat-Ionen und/oder Basen, die mit Mikroorganismen der Kultur und/oder Mischungen davon physiologisch kompatibel sind.

5. Verfahren nach Anspruch 4, wobei der Schritt der pH-Kontrolle durch Zugabe von Ammoniak und/oder Ammoniumhydroxid oder Carbonat-Ionen in ein- und/oder zweibasischen Formen zu dem Nährmedium für das Wachstum des Bakterienstammes bzw. der Bakterienstämme erfolgt.

6. Verfahren nach Anspruch 1, wobei der pH-Endwert der Kultur zwischen 5,0 und 5,2 liegt.

7. Verfahren nach einem der vorhergehenden Ansprüche, wobei:
- von den Stämmen der Gattung *Lactobacillus* die folgenden Arten ausgewählt werden: *L. pentosus, L. plantarum, L. casei, L. casei ssp. paracasei, L. casei* ssp. *rhamnosus, L. acidophilus, L. delbrueckii* ssp. *bulgaricus, L. fermentum, L. gasseri;*
- von den Stämmen der Gattung *Bifidobacterium* die folgenden Arten ausgewählt werden: *B. longum, B. breve, B. lactis, B. adolescentis, B. pseudocatenulatum, B. catenulatum;*
- von den Stämmen der Gattung *Lactococcus* die folgenden Arten ausgewählt werden: *L. lactis* und *L. lactis* ssp. *lactis;*
- von den Stämmen der Gattung *Streptococcus* die folgende Art ausgewählt wird: *S. thermophilus.*

## Revendications

1. Méthode de préparation d'une culture de départ liquide comprenant au moins un micro-organisme physiologiquement compatible, **caractérisée en ce que** la concentration cellulaire dudit micro-organisme dans ladite culture de départ liquide est >10⁹ CFU/ml de milieu de culture, **en ce que** ladite culture de départ liquide a une durée de conservation de 6 jours ou plus, à une température moyenne de 3 à 5 °C et **en ce qu'**elle est basiquement exempte de sucres résiduaires, dans laquelle ladite méthode consiste à
i) inoculer un milieu de culture avec une quantité efficace de culture mère constituée d'au moins une souche bactérienne, ou d'un mélange de souches ;
ii) attendre le début de la fermentation et le début du stade de croissance exponentielle de la biomasse bactérienne ;
iii) contrôler le pH du milieu de culture, en maintenant ladite valeur dans une plage de 4,5 à 7,0, au moyen d'ajouts progressifs d'au moins une base ou d'un mélange de plusieurs bases, pendant le temps requis pour que de 2 à 5 duplications cellulaires de la ou des souches se produisent ;
iv) laisser le pH du milieu de culture descendre spontanément jusqu'à une valeur de 4,7 à 5,6, pendant un laps de temps suffisant pour qu'une nouvelle duplication cellulaire se produise ;
v) refroidir la culture obtenue à l'étape iv) jusqu'à une température de 4 à 8 °C ;
dans laquelle ledit au moins un micro-organisme est choisi dans le groupe des souches microbiennes de l'un des genres suivants : *Lactobacillus, Leuconostoc, Bifidobacterium, Lactococcus, Pediococcus, Streptococcus* et *Enterococcus.*

2. Méthode selon la revendication 1, dans laquelle à l'étape iii) pendant ladite étape de contrôle, la valeur de pH est maintenue dans une plage de 5,6 à 6,2.

3. Méthode selon la revendication 2, dans laquelle à l'étape iii), la valeur de pH est maintenue dans une plage de 5,7 à 6,0.

4. Méthode selon l'une quelconque des revendications 1 à 3, dans laquelle ladite au moins une base est choisie dans le groupe comprenant : un ion carbonate, sous ses formes mono- et/ou dibasiques, l'ammoniac, des hydroxydes, p. ex. hydroxydes d'ammonium, de sodium, de potassium, un ion phosphate, sous ses formes mono- et/ou di- et/ou tribasiques, un ion sulfate, sous ses formes mono- et/ou dibasiques, des ions citrate, un ion tartrate, et/ou des bases qui sont physiologiquement compatibles avec les micro-organismes de la culture, et/ou des mélanges de ceux-ci.

5. Méthode selon la revendication 4, dans laquelle ladite étape de contrôle du pH est mise en oeuvre par ajout au milieu de culture pour la croissance de la ou des souches bactériennes d'ammoniac et/ou d'un hydroxyde d'ammonium, ou d'un ion carbonate, sous ses formes mono- et/ou dibasiques.

6. Méthode selon la revendication 1, dans laquelle le pH final de la culture est de 5,0 à 5,2.

7. Méthode selon l'une quelconque des revendications précédentes, dans laquelle :
- lesdites souches du genre *Lactobacillus* sont choisies parmi les espèces suivantes : *L. pentosus, L. plantarum, L. casei, L. casei* ssp. *paracasei, L. casei* ssp. *rhamnosus, L. acidophilus, L. delbrueckii* ssp. *bulgaricus, L. fermentum, L. gasseri ;*
- lesdites souches du genre *Bifidobacterium* sont choisies parmi les espèces suivantes : *B. longum, B. breve, B. lactis, B. adolescentis, B. pseudocatenulatum, B. catenulatum ;*
- lesdites souches du genre *Lactococcus* sont choisies parmi les espèces suivantes : *L. lactis* et *L. lactis* ssp. *lactis ;*
- lesdites souches du genre *Streptococcus* sont choisies parmi les espèces *S. thermophilus*.
